# EUROPÄISCHE PATENTANMELDUNG

(11) **EP 1 623 727 A1**
(43) Veröffentlichungstag der Anmeldung: **08.02.2006**
(21) Anmeldenummer: 04405451.8
(22) Anmeldetag: 14.07.2004
(51) Int. Cl.: A61L 27/50, A61L 27/36, A61F 2/28, A61F 2/44

(54) **Füllmittel und Zuführvorrichtung zum Ausbilden einer Stützstruktur in einem Knocheninnenraum**

(71) Anmelder: Sidler, Bruno, 5737 Menziken (CH)
(72) Erfinder: Sidler, Bruno, 5737 Menziken (CH)
(74) Vertreter: Dr. Graf & Partner

(57) **Zusammenfassung**

Das trockenfliessfähiges Füllmittel (1) zum Ausbilden einer Stützstruktur in einem Knocheninnenraum (4a), umfassend eine Mehrzahl biokompatibler Stützkörper (2), welche unter den üblicherweise im Knocheninnenraum (4a) auftretenden physiologischen Lasten resistent gegen Verformung oder Bruch sind, wobei die Stützkörper (2) eine Grösse zwischen 2 mm und 10 mm aufweisen.

## Beschreibung

Die vorliegende Erfindung betrifft ein Füllmittel zum Ausbilden einer Stützstruktur in einem Knocheninnenraum gemäss dem Oberbegriff von Anspruch 1. Die Erfindung betrifft weiter eine Zuführvorrichtung zum Einfüllen eines Füllmittels in einen Knocheninnenraum gemäss dem Oberbegriff von Anspruch 17. Die Erfindung betrifft weiter ein Verfahren zum Füllen eines Knocheninnenraumes mit einer Stützstruktur gemäss dem Oberbegriff von Anspruch 21.

Die Erfindung bezieht sich auf die Behandlung von Knochen bei Menschen oder Tieren.

Die zunehmende Alterung der Gesellschaft führt zu einer überdurchschnittlichen Zunahme von Erkrankungen des Bewegungs- und Stützapparates, insbesondere der Knochen. Knochen können auf unterschiedlichste Arten geschädigt oder geschwächt werden, beispielsweise durch Trauma, Infektion, Abnutzung, Tumorwachstum oder degenerative Krankheiten wie Osteoporose. Bei älteren Menschen stellt insbesondere die Osteoporose, das heisst der Abbau der Spongiosa, ein Problem dar, weil dadurch die Belastungsfähigkeit des Knochens geschwächt wird, was zur Folge hat, dass vermehrt Knochenbrüche auftreten, vor allem an der Wirbelsäule, am Schenkelhals und am Handgelenk. Eine Behandlung derartiger Knochenbrüche ist schwierig, insbesondere wenn degenerative Veränderungen im fortgeschrittenen Stadium vorliegen. Zur Fixation derartiger Knochenbrüche werden üblicherweise äussere oder innere Schienungen (Platte, Schrauben, Implantate) verwendet, welche den Knochen bis zu dessen Heilung zusammenhalten. Derartige Schienen sind jedoch nicht bei allen Knochen verwendbar. So wird beispielsweise bei der Wirbelsäule zur Behandlung eines degenerierten oder eingebrochenen Wirbelkörpers die sogenannte Vertebroplastie angewandt, indem der geschädigte Wirbelkörper mit Knochenzement gefüllt wird. Die Verwendung von Knochenzement bei Wirbelkörpern weist jedoch verschiedene Nachteile auf, insbesondere dass der Knochenzement über Venen oder kleine Knochendefekte im Wirbelkörper unkontrolliert austreten kann, und dadurch Schäden in den benachbarten anatomischen Strukturen verursachen kann, beispielsweise bei einem Austritt in den Spinalkanal. Weitere Nachteile von Knochenzement sind, dass sich dieser beim Aushärten stark erwärmt, was umliegendes Gewebe oder sogar Nerven schädigen kann, dass der Knochenzement sehr schnell verarbeitet werden muss, dass im Knochenzement kein Knochen nachwachsen kann, und dass der Knochenzement mit der Zeit spröde wird.

Die Druckschrift US 2004/0052829 offenbart ein Verfahren zur Behandlung poröser Wirbelkörper, insbesondere von Wirbelkörpern mit Osteoporosefraktur. Dazu wird eine biokompatible, flüssige Trägersubstanz wie Wasser verwendet, welche mit biokompatiblen Stützkörpern angereichert ist. Nachteilig an diesem Verfahren ist die Tatsache, dass ein ballonartiger Behälter erforderlich ist, welcher zuerst in den Wirbelkörper einzubringen ist, und welcher danach mit der Trägersubstanz enthaltend die Stützkörper gefüllt wird. Der ballonartige Behälter ist erforderlich um sicherzustellen, dass sowohl die flüssige Trägersubstanz als auch die darin enthaltenen Stützkörper innerhalb des Wirbelkörpers verbleiben. Würde kein ballonartiger Behälter verwendet, so bestünde auch bei diesem Verfahren die bekannte Gefahr des Ausfliessens, indem die flüssige Trägersubstanz sowie die darin enthaltenen Stützkörper, welche eine Grösse im Mikrometerbereich aufweisen, aus dem geschädigten Wirbelkörper entweichen, und auf unkontrollierbare Art benachbartes Gewebe schädigen oder sich im menschlichen Körper ausbreiten. Das bekannte Verfahren weist somit die Nachteile auf, dass es aufwendig ist den ballonartigen Behälter in den Wirbelkörper einzubringen, dass dieser beim Einbringen oder durch die Stützkörper beschädigt werden kann, sodass die Trägersubstanz ausfliessen könnte, und dass der Wirbelkörper nicht optimal füllbar ist, da sich der ballonartige Behälter während dem Zuleiten der flüssigen Trägersubstanz "aufbläst", und somit mehr Volumen als für die Stützkörper an sich erforderlich beansprucht.

Es ist daher Aufgabe der vorliegenden Erfindung ein vorteilhafteres, implantierbares, insbesondere injizierbares Füllmittel vorzuschlagen, welches defekte Knochen, insbesondere Wirbelkörper, derart optimal zu versorgen erlaubt, dass die Knochen vom Zeitpunkt der Implantation an fähig sind die anliegenden physiologischen Lasten zu tragen.

Diese Aufgabe wird gelöst mit einem trockenfliessfähigen Füllmittel aufweisend die Merkmale von Anspruch 1. Die Unteransprüche 2 bis 17 betreffen weitere vorteilhaft ausgestaltete Füllmittel. Die Aufgabe wird weiter gelöst mit einer Zuführvorrichtung zum Einführung des Füllmittels aufweisend die Merkmale von Anspruch 18. Die Unteransprüche 19 und 20 betreffen weitere vorteilhafte Ausgestaltungen. Die Aufgabe wird weiter gelöst mit einem Verfahren zum Füllen eines Knocheninnenraumes mit Stützkörpern aufweisend die Merkmale von Anspruch 21. Die Unteransprüche 22 bis 26 betreffen weitere, vorteilhafte Verfahrensschritte.

Die Aufgabe wird insbesondere gelöst mit einem trockenfliessfähigen Füllmittel zum Ausbilden einer Stützstruktur in einem Knocheninnenraum, wobei das Füllmittel eine Mehrzahl biokompatibler Stützkörper umfasst, welche unter den üblicherweise im Knocheninnenraum auftretenden physiologischen Lasten resistent gegen Verformung oder Bruch sind, wobei die Stützkörper eine Grösse zwischen 2 mm und 10 mm aufweisen.

Der Ausdruck "trockenfliessfähig" bedeutet, dass das Füllmittel injizierbar ist, jedoch ohne Verwendung irgendeiner fluiden Trägersubstanz, welche den Stützkörpern Fliesseigenschaften verleihen könnte. Die Stützkörper sind derart gross gewählt, dass diese, zum Beispiel in einer Kanüle hintereinander liegend angeordnet, alle gemeinsam in der Kanüle verschiebbar sind, indem auf den hintersten Stützkörper ein Druck ausgeübt wird, und diese Kraft auf alle sich in der Kanüle befindlichen Stützkörper übertragen wird, sodass die Stützkörper in der Kanüle zur Kanülenspitze hin bewegt werden. Diese Stützkörper weisen trockenfliessfähige Eigenschaften auf, indem diese in der Kanüle ohne eine Trägersubstanz eine Art fliessende Eigenschaften aufweisen, indem die Stützkörper, ähnlich einer Injektion, über die Kanüle einem Knocheninnenraum zuführbar sind.

Der Ausdruck "Stützkörper" bezeichnet einen Körper, welcher die in einem Knocheninnenraum, insbesondere die in einem Wirbelkörper auftretenden Kräfte zu tragen vermag, ohne wesentlich verformt oder gar zerstört zu werden. Es sind eine Vielzahl von biokompatibler Werkstoffe bekannt, aus welchen ein derartiger Stützkörper gefertigt sein kann. Beispielsweise könnte der Stützkörper gefertigt sein aus:
- keramischen Werkstoffen, insbesondere Calciumphosphate /Hydroxylapatite, Aluminiumoxide, Zirkoniumoxide, ATZ Geramk (Alu-Zirkonium-Oxid), bioaktive Gläser, Glaskeramiken, Porzellan oder einer Kombination davon, oder
- metallischen Werkstoffen, insbesondere Titan, Tantal, rostfreier Stahl, Stahllegierungen wie Kobalt-Chrom Legierung, Titanlegierungen wie Titan-Nickel Legierung oder Titan-Aluminium-Niobium/Vanadium Legierung, oder einer Kombination davon, oder
- Polymeren, insbesondere Polymethylmethacryl (PMMA), Polyetheretherketon (PEEK) Polyethylen (PE), Polyethylenterephthalat (PET), oder einer Kombination davon, oder
- biodegradablen Polymeren wie Polylactate.

Das erfindungsgemässe Füllmittel umfassend eine Mehrzahl biokompatibler Stützkörper weist die Vorteile auf, dass kein Ballon oder ein anderes Begrenzungsmittel im Knocheninnenraum erforderlich ist, da das Füllmittel einerseits keine Trägersubstanz wie eine Flüssigkeit aufweist, welche auslaufen könnte, und da das Füllmittel andererseits aus einzelnen, relativ grossen Stützkörpern besteht, welche auf Grund ihrer Grösse auch einem stark geschädigten Wirbelkörper kaum unkontrolliert entwichen können, und sich zudem auf Grund ihrer Grösse nicht unkontrolliert im Körper verteilen können. Das erfindungsgemässe Füllmittel ist trockenfliessfähig und kann daher mit Hilfe einer Kanüle in einen Knocheninnenraum injiziert werden. Das erfindungsgemässe Füllmittel ist daher insbesondere auch zur Versorgung schwer zugänglicher Knochen wie der Wirbelknochen geeignet.

In einer besonders vorteilhaften Ausführungsform sind die Stützkörper derart ausgestaltet, dass sie sich gegenseitig verkeilen können, sodass die sich im Knocheninnenraum befindlichen Stützkörper gegenseitig verkeilen und dadurch eine zusammenhängende Stützstruktur bilden.

Das erfindungsgemässe Füllmittel erlaubt den Knochendefekt frakturierter Knochen, insbesondere Wirbelkörper, mit einer Stützstruktur bestehend aus einzelnen Stützkörpern zu füllen. Diese Stützstruktur verleiht dem Knochen eine Stabilität, überträgt auftretende Kräfte, und kann zudem die Knochenheilung und/oder die Knochenbildung anregen. Die Stützkörper können zudem mit knochenheilenden und/oder knochenbildenden Substanzen gefüllt und/oder beschichtet sein oder eine knochenheilende und/oder knochenbildende Oberflächenstruktur aufweisen. Das erfindungsgemässe Füllmittel ist mittels einer kleinen Kanüle in den Knochen einbringbar, was einen schonenden Zugang zum Knochen ermöglicht.

Die Erfindung wird nachfolgend an Hand von Figuren im Detail beschrieben. Es zeigen:
- Fig. 1: eine Aufsicht auf einen Wirbelkörper mit einer eingeführten Kanüle;
- Fig. 2: einen Schnitt durch einen Wirbelkörper, in welchen das Füllmittel eingeführt wird;
- Fig. 3: einen Schnitt durch den Wirbelkörper sowie die Kanüle gemäss Fig. 2;
- Fig. 4: einen Schnitt durch den Wirbelkörper mit vollständig eingeführtem Füllmittel;
- Fig. 5a bis 5e: schematische Darstellungen unterschiedlich ausgebildeter Stützstrukturen;
- Fig. 6a bis 6c: Stützkörper mit eckigen Aussenkonturen;
- Fig. 7a bis 7c: Stützkörper mit abgerundeten Aussenkonturen;
- Fig. 8a bis 8d: Stützkörper mit polyederförmigen Aussenkonturen;
- Fig. 9: ein weiteres Ausführungsbeispiel eines teilweise mit Füllmittel gefüllten Knochenhohlraumes;
- Fig. 10: das Befestigen einer Kanüle in einem Knochen;
- Fig. 11: eine Kanüle;
- Fig. 12: eine Kanülenspitze;
- Fig. 13: eine Pressvorrichtung;
- Fig. 14: ein gebrochener Knochen mit Knochenhohlraum;
- Fig. 15: ein Querschnitt durch eine Kanüle;
- Fig. 16: ein weiterer Querschnitt durch eine Kanüle.

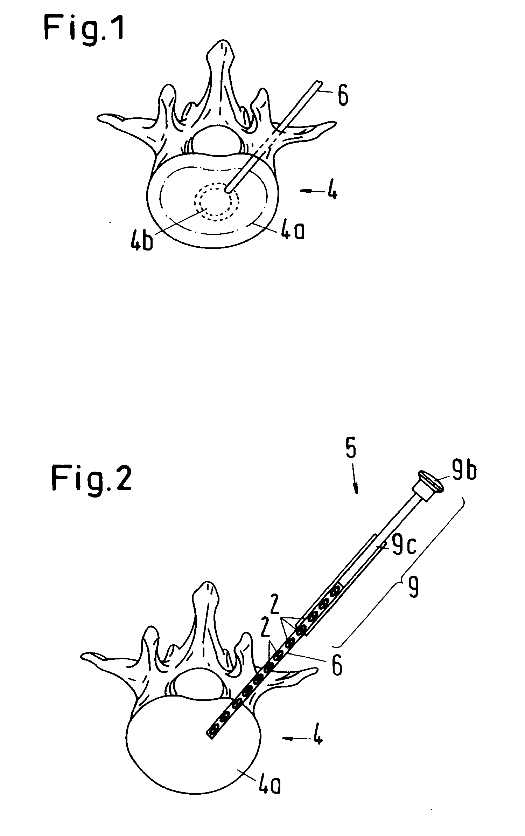

Figur 1 zeigt in einer Aufsicht einen Knochen 4, spezifischer einen Wirbelkörper mit einem Knocheninnenraum 4a. Unter dem Begriff Knocheninnenraum 4a wird das gesamte, vom Knochen 4 eingenommene Volumen verstanden. Im Knocheninnenraum 4a befindet sich ein Knochenhohlraum 4b, in welchen eine Kanüle 6 mündet. Der Knochenhohlraum 4b kann beispielsweise auf Grund degenerativer Vorgänge, insbesondere Osteoporose, entstanden sein. Der Knochenhohlraum 4b kann mit einem geeigneten Instrument auch künstlich erzeugt oder vergrössert werden.

Figur 2 zeigt einen Wirbelkörper 4 mit einem Knocheninnenraum 4a sowie eine Zuführvorrichtung 5 umfassend eine Kanüle 6, eine Pressvorrichtung 9 mit Betätigungsgriff 9b und einem Stössel 9c. Die Kanüle 6 münden in den Knocheninnenraum 4a, welcher in diesem Ausführungsbeispiel keinen Knochenhohlraum 4b aufweist. Im Innenraum der Kanüle 6 und der Pressvorrichtung 9 sind biokompatible Stützkörper 2 hintereinander liegend und sich im wesentlichen gegenseitig berührend angeordnet, sodass der auf den zuhinterst liegenden Stützkörper 2 wirkende Stössel 9c alle Stützkörper 2 in Richtung Knocheninnenraum 4a treibt. Dies ermöglicht, falls erforderlich, über die Stützkörper 2 die am Betätigungsgriff 9b anliegende Kraft bis zu den sich an der Spitze der Kanüle 6 befindlichen Stützkörpern 2 zu übertragen, sodass diese mit einer entsprechenden Kraft in den Knocheninnenraum 4a eindringen, und dabei während dem Eindringen einen Knochenhohlraum 4b ausbilden. Der Innendurchmesser der Kanüle 6 sowie der Pressvorrichtung 9 sind bezüglich dem Aussendurchmesser der Stützkörper 2 derart angepasst ausgestaltet, dass die Stützkörper 2 in Flussrichtung, d.h. zur Austrittsöffnung der Kanüle 6 hin, hintereinanderliegend angeordnet sind, was zur Folge hat, dass die Gesamtheit der Stützkörper 2 ein trockenfliessfähiges Füllmittel 1 bilden, welches ohne jegliche Schmiermittel fliessähnliche Eigenschaften aufweist, indem die Stützkörper 2 mit einer wie in Figur 2 dargestellt spritzenähnlichen Vorrichtung dem Knocheninnenraum 4a zuführbar ist. Die Kanüle 6 könnte auch gekrümmt verlaufen, oder aus einem flexiblen oder festen Material bestehen.

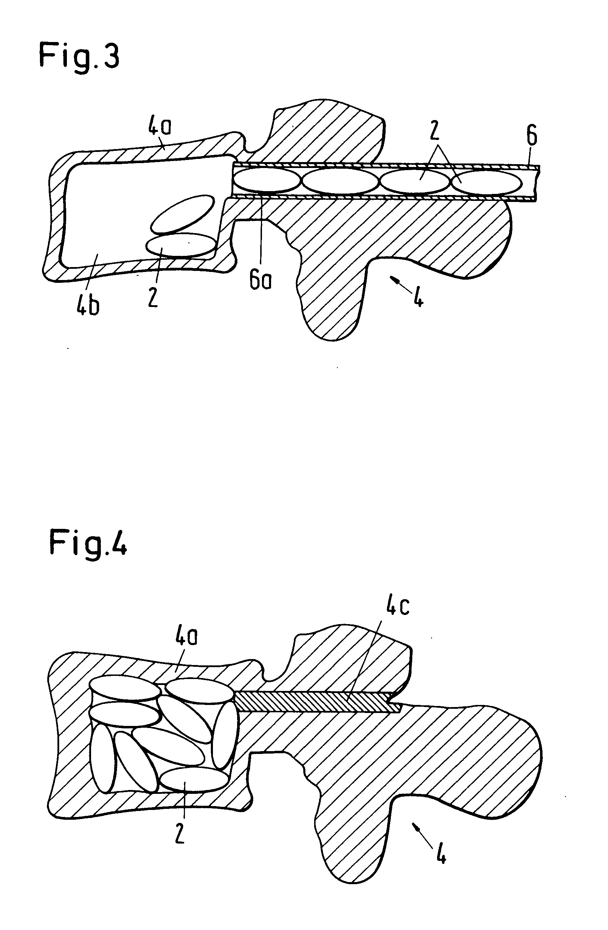

Der in Figur 3 in einem Schnitt dargestellte Wirbelkörper 4 weist einen Knochenhohlraum 4b auf, welcher vorgängig, vor dem Einführen der Stützkörper 2, mit einem speziellen Instrument erzeugt wurde. Danach wird die Spitze 6a der Kanüle 6 bis zum Knochenhohlraum 4b vorgetrieben, und danach die in einer Seitenansicht dargestellten Stützkörper 2 einzeln und nacheinander folgend zugeführt, sodass sich diese zufällig im Knochenhohlraum 4b verteilen. Die Kanüle 6 weist einen runden Innenquerschnitt auf, sodass jeder Stützkörper 2, welche alle identisch ausgestaltet sind, eine längliche, sphärisch verlaufende Aussenkontur aufweist. Der Stützkörper 2 weist eine maximale Dimension zwischen 2 mm und 10 mm auf. Die Stützkörper 2 sind als Vollkörper ausgestaltet. Alle Stützkörper 2 sind betreffend Dimension und Form identisch ausgestaltet.

Figur 4 zeigt den in Figur 3 dargestellten Wirbelkörper 4 mit einem vollständig durch Stützkörper 2 gefüllten Knochenhohlraum 4b, wobei die Stützkörper 2 an unterschiedlichen Stelle aufeinander aufliegen und dabei im Knochenhohlraum 4b gefangen sind, sodass die Gesamtheit dieser Stützkörper 2 eine tragfähige Stützstruktur bilden. Der Zugangskanal zum Knochenhohlraum 4b wurde nach dem Einführen der Stützkörper 2 mit einem Zapfen 4c verschlossen.

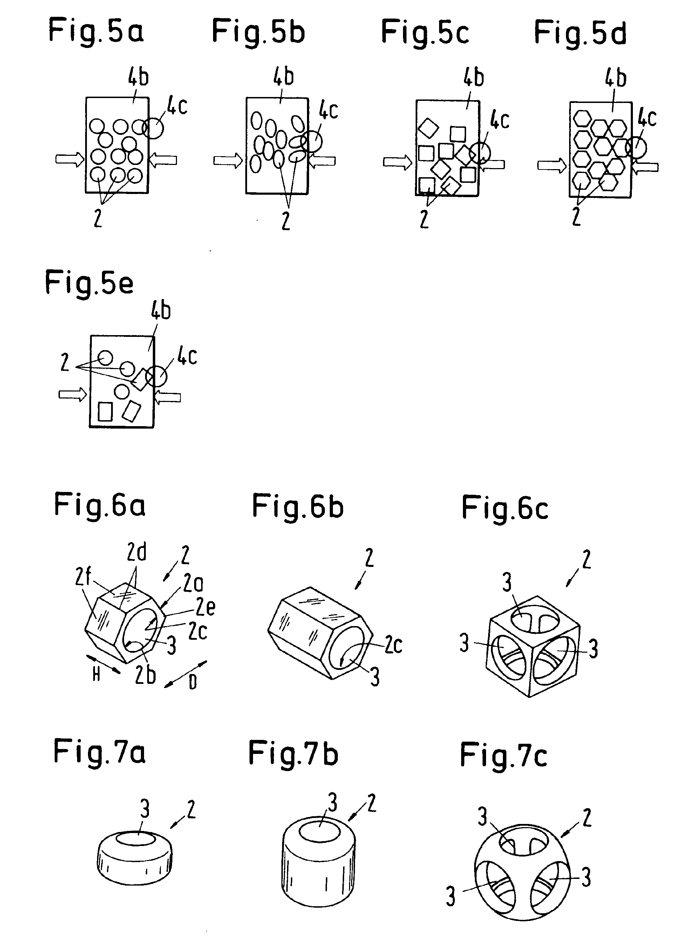

Ein Knocheninnenraum 4a beziehungsweise ein Knochenhohlraum 4b kann mit unterschiedlichst ausgestalteten Stützkörpern 2 gefüllt werden. Figur 5a zeigt schematisch einen begrenzten Knochenhohlraum 4b mit Einlassöffnung 4c, durch welche die kugelförmigen Stützkörper 2 eingeführt werden. Figur 5b zeigt ellipsoidförmig ausgestaltete Stützkörper 2. Ein Vorteil der in den Figuren 5a und 5b dargestellten Stützkörpern 2 mit sphärischer Aussenkontur ist die Tatsache, dass sie unter geringem Kraftaufwand gegenseitig verschiebbar sind, sodass diese Stützkörper 2 das Volumen des Knochenhohlraumes 4b sehr gut ausfüllen. Die Figuren 5c und 5d zeigen Stützkörper 2 mit einer kantigen Aussenkontur, wobei die unter einem Winkel zusammenlaufenden Seitenflächen der Stützkörper 2 eine Keilwirkung ausüben können, sodass sich die im begrenzten Knochenhohlraum 4b befindlichen Stützkörper 2 in einer vorteilhaften Ausführungsform, wie in Figur 5c dargestellt, gegenseitig verkeilen, wobei sich zwischen dem Stützkörper 2 grössere Zwischenräume ergeben, in welche der Knochen nachwachsen kann. Die Gesamtheit der Stützkörper 2 bilden wiederum eine mechanisch belastbare Stützstruktur. Im Gegensatz zu den in den Figuren 5a bis 5d dargestellten Stützkörpern 2, welche jeweils bezüglich ihrer Form und Grösse identisch sind, könnten die Stützkörper 2, wie in Figur 5e dargestellt, innerhalb des Knochenhohlraumes 4b auch unterschiedliche Formen und/oder Grössen aufweisen.

Die Figuren 6a, 6b und 6c sowie 7a, 7b und 7c zeigen Stützkörper 2 mit einem offenen Innenhohlraum 3. Unter einem offenen Innenhohlraum 3 wird ein Hohlraum im Stützkörper 2 verstanden, welcher gegen Aussen offen ist, im Gegensatz zu einem geschlossenen Hohlraum, welcher vollständig im Innern des Stützkörpers 2 angeordnet ist, ohne eine Öffnung nach Aussen aufzuweisen. Auch diese Stützkörper 2 weisen eine maximale Grösse im Bereich zwischen 2 mm und 10 mm auf. Jeder Stützkörper 2 umfasst ein Gesamtvolumen, welches dem Volumen des Materials des Stützkörpers 2 sowie dessen Innenhohlraum 3 entspricht. Das Volumen des Innenhohlraumes ist grösser als 30% des Gesamtvolumens, vorzugsweise grösser als 50% und kann bis zu 90% betragen. Die Grösse des maximal möglichen Volumens des Innenhohlraumes 3 ist abhängig von den maximal am Stützkörper 2 angreifenden Druckkräften. Diese Druckkräfte sind abhängig vom spezifischen Knocheninnenraum 4a beziehungsweise von Knochen 4, in welchem die Stützstruktur gebildet wird. Die Tragfähigkeit des Stützkörpers 2 ist natürlich abhängig vom verwendeten Material. Wird der Stützkörper 2 beispielsweise aus Metall gebildet, so kann der Innenhohlraum 3 relativ gross ausgebildet sein, und der Stützkörper 2 trotzdem resistent gegen die angreifenden Druckkräfte sein. Ist der Stützkörper 2 aus einem Material wie Bioglas oder einer resorbierbaren Substanz gefertigt, so muss der Innenhohlraum 3 entsprechend den Materialeigenschaften prozentual kleiner ausgebildet sein, um dem Stützkörper 2 eine genügend grosse Tragkraft zu verleihen. Dieser Innenhohlraum 3, insbesondere ein prozentual relativ grosser Innenhohlraum 3, weist den Vorteil auf, dass dieser mit der Zeit von nachwachsendem Knochenmaterial gefüllt werden kann. In einer besonders vorteilhaften Ausgestaltung wird der Innenhohlraum 3 des Stützkörpers 2 vor dessen Einführen zumindest teilweise mit einer osteoinduktiven und/oder osteokonduktiven Substanz gefüllt, insbesondere einem knochenwachstumsfördernden Protein oder Kalziumsulfat oder einer Kombination dieser oder weiterer Substanzen. Dank derart gefüllten Stützkörpern 2 ist es möglich dem Knocheninnenraum 4a mittels dem trockenfliessfähigen Füllmitteln 1, bestehend aus einer Vielzahl von Stützkörpern 2, sowohl eine Stützstruktur als auch osteoinduktive und/oder osteokonduktive Substanzen zuzuführen. Diese Ausgestaltung weist die Vorteile auf, dass das Füllmittel 1 trocken dem Knocheninnenraum 4a zugeführt wird, sodass keine Gefahr eines Auslaufens durch eventuell im Knochen 4 vorhandene Ritzen, Spalten und Öffnungen besteht. Zudem bewirkt die osteoinduktive Substanz ein Knochenwachstum, sodass die Hohlräume 3 und die sich zwischen den Stützkörpern 2 ergebenden Zwischenräume vorteilhafterweise zunehmend mit nachwachsendem Knochen gefüllt werden. Da die Stützkörper 2 im Knocheninnenraum 4a zufällig ausgerichtet angeordnet sind, das heist die Hohlräume 3 in zufälligen Richtungen verlaufen, und auch die Zwischenräume in zufälligen Richtungen verlaufen und eine durch den Zufall bestimmte Grösse aufweisen, bilden die Stützkörper 2 an sich, insbesondere wenn das Volumen des Innenhohlraumes 3 mehr als 50% des Gesamtvolumens beträgt, eine im weitesten Sinne Spongiosa ähnliche Stützstruktur. Wenn mit zunehmendem Heilungsverlauf zudem die Hohlräume 3 und die Zwischenräume mit nachwachsender Spongiosa gefüllt werden, so bildet sich im Knocheninnenraum 4a eine dem gesunden Knochen teilweise vergleichbare Stützstruktur aus, mit willkürlich ausgerichteten Stützkörpern 2, deren Hohl- und Zwischenräume mit Spongiosa verwachsen sind.

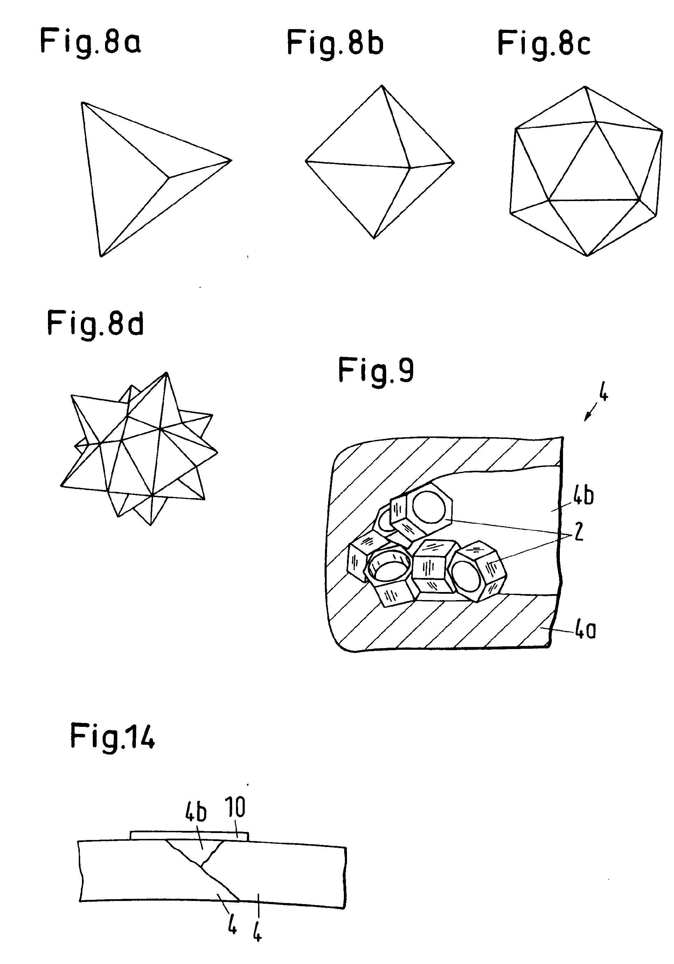

Der Innenhohlraum 3 der Stützkörper 2 gemäss der Figuren 6a,6b,6c,7a,7b,7c ist zusammenhängend und nicht porös ausgestaltet, sodass die Innenkontur 2b seitlich einen relativ grossen Innenhohlraum 3 begrenzt. Der Innenhohlraum 3 gemäss der Figuren 6a,6b,7a,7b ist zylinderförmig ausgestaltet und weist eine kreisförmige Öffnung 2c auf. Die Stützkörper 2 gemäss den Figuren 6a, 7a sind im wesentlichen ringförmig ausgestaltet, wobei die Ausführungsform gemäss Figur 6a in Umfangsrichtung eine polyederförmige Aussenkontur bestehend aus sechs gegenseitig keilförmig verlaufenden Oberflächen 2f aufweist, welche sich jeweils in einer Kante 2d bzw. Ecke 2e treffen. Der Stützkörper 2 weist in Umfangsrichtung vorzugsweise eine 3- bis 10-eckige Aussenkontur auf, insbesondere eine 4-, 5- oder 6-eckige Aussenkontur. Die Ausführungsform gemäss Figur 7a weist in Umfangsrichtung eine sphärisch, insbesondere kugelförmig oder ellipsoidförmig verlaufende Aussenkontur auf. Die Stützkörper 2 gemäss den Figuren 6b und 7b sind im wesentlichen hohlzylinderförmig ausgestaltet. Die Stützkörper 2 gemäss den Figuren 6c und 7c weisen im wesentlichen eine würfelförmige bzw. sphärisch verlaufende Aussenkontur auf. Die Figuren 8a bis 8d zeigen weitere Ausführungsform von Stützkörpern 2 mit polyederförmig verlaufenden Aussenkonturen auf, wobei die dargestellten Stützkörper 2 keinen Hohlraum 3 aufweisen. Die Stützkörper 2 könnten jedoch auch einen Hohlraum 3 aufweisen. Figur 8a zeigt ein Tetraeder, Figur 8b ein Oktaeder, Figur 8c ein Ikosaeder und Figur 8d ein kleines Sternendodekaeder.

In einer vorteilhaften Ausgestaltung weist der Stützkörper 2, wie in Figur 6a dargestellt einen Aussendurchmesser D und eine dazu orthogonale Höhe H auf, wobei der Aussendurchmesser D vorzugsweise zumindest das 1,5-fache der Höhe H beträgt. Die Stützkörper 2 weisen in einer vorteilhaften Ausgestaltung eine derart ausgebildete Oberfläche auf, dass zwischen den im Knocheninnenraum 4a eingefüllten Stützkörpern 2 ein gegenseitiges Verkeilen beziehungsweise Verklemmen auftritt, um innerhalb des Knocheninnenraumes 4a eine vorzugsweise zusammenhängende Stützstruktur zu bilden. Die Oberfläche der Stützkörper 2 weist daher vorzugsweise Formen aus der Gruppe Ecke, Kante, Spitze, Vertiefung, Durchbrechung oder eine Kombination davon auf, um ein gegenseitiges Verkeilen und Verklemmen der Stützkörper 2 zu bewirken. In einer vorteilhaften Ausgestaltung weist der Stützkörper 2, wie in Figur 6a dargestellt, eine Schraubenmutter ähnliche Form auf, mit einer mehreckigen Aussenkontur mit gegenseitig keilförmig verlaufenden Flächen 2f und markanten Kanten 2d und Ecken 2e, sowie einem Innenhohlraum 3 mit einer Öffnung 2c mit relativ grossem Durchmesser. Figur 9 zeigt einen erst teilweise mit derartigen, schraubenmutterförmigen Stützkörpern 2 gefüllten Knochenhohlraum 4b. Aus dieser Anordnung der Stützkörper 2 ist ersichtlich, wie sie sich gegenseitig verkeilen, indem die Aussenkontur über die Öffnung 2c teilweise in den Innenhohlraum 3 eindringt, sodass sich die Stützkörper 2 gegenseitig verkeilen, und derart eine mechanisch zumindest zusammenhängende Stützstruktur ausbilden. Sobald der gesamte Knochenhohlraum 4b vollständig mit diesen Stützkörpern 2 gefüllt ist, kann die derart ausgebildete Stützstruktur die von Aussen auf den Knochen 4 einwirkenden Last im wesentlichen als Verbund tragen.

Figur 14 zeigt einen gebrochenen Knochen 4, wobei an der Bruchstelle ein Knochenteil fehlt. Diese Stelle ist mit einer Knochenplatte 10 abgedeckt, sodass sich ein Knochenhohlraum 4b ausbildet. Auch ein derartiger Knochenhohlraum 4b kann mit dem oben beschriebenen Füllmittel 1 umfassend Stützkörper 2 gefüllt werden.

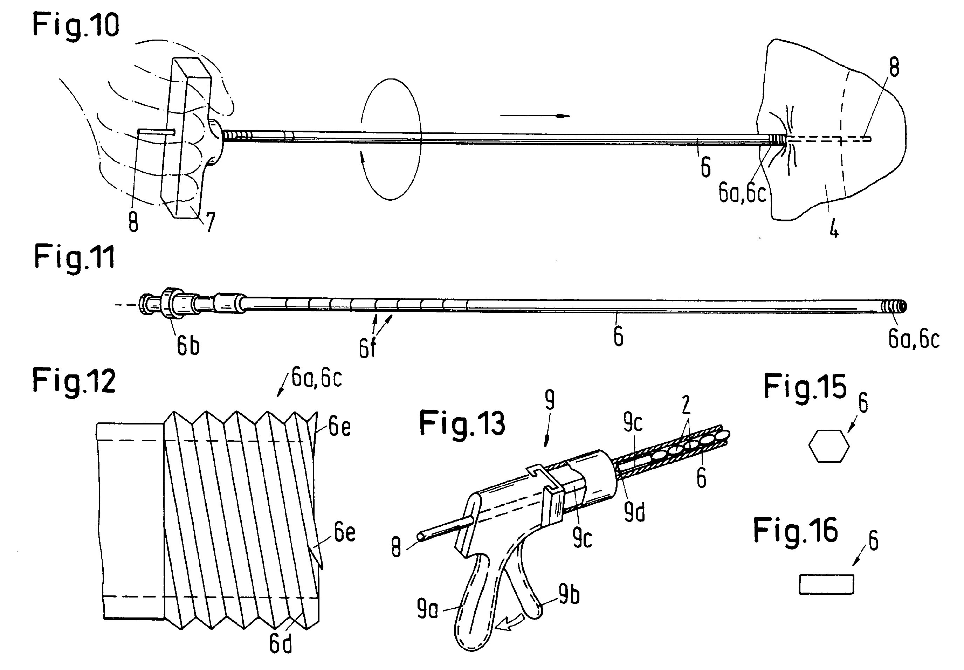

Figur 10 zeigt eine feste Kanüle 6 mit einer als Schneidgewinde 6c ausgestalteten Kanülenspitze 6a. Innerhalb der Kanüle 6 verläuft ein Kirschnerdraht 8. Vorerst wird der Kirschnerdraht 8 in den Knochen 4 gestossen, um die Verlaufsrichtung der Kanüle 6 zu bestimmen. Danach wird die Spitze 6a der Kanüle 6 unter Verwendung des Handgriffes 7 mit einer rotierenden Bewegung in den Knochen 4 geschraubt, sodass die Spitze 6a und somit die Kanüle 6 fest im Knochen 4 verankert ist. Danach wird der Handgriff 7 abgenommen. Figur 11 zeigt die Kanüle 6 mit entferntem Handgriff 7. Die Kanüle 6 umfasst ein Anschlussteil 6b für den Handgriff 7 beziehungsweise für eine Pressvorrichtung 9. Figur 12 zeigt ein Ausführungsbeispiel einer Spitze 6a der Kanüle 6 im Detail. Die Spitze 6a weist vorstehende Schneidkörper 6e und ein Aussengewinde 6d auf. Figur 13 zeigt eine Pressvorrichtung 9, welche über das Anschlussteil 6b mit der Kanüle 6 verbunden ist. Vor dem Verbinden wurde die Kanüle 6 mit Stützkörpern 2 gefüllt. Die Pressvorrichtung 9 umfasst einen Handgriff 9a sowie einen Betätigungsgriff 9b, welcher derart auf einen Stössel 9c wirkt, dass dieser sich bei jeder Betätigung ein Stück weit in die Kanüle 6 hineinbewegt, sodass der auf die Stützkörper 2 wirkende Stössel 9c die in der Kanüle 6 zuvorderst angeordneten Stützkörper 2 in den Knochen 4 presst. Die Stützkörper 2 fliessen derart in den Knochen 4 hinein, bilden darin auf Grund des Druckes zudem einen Knochenhohlraum 4b aus, und füllen diesen mit Stützkörpern 2, sodass innerhalb des Knochens 4 eine Stützstruktur ausgebildet wird. Die Lage des Stössels 9c kann mit Hilfe von an der Kanüle 6 angebrachten Markierungen 6f abgelesen werden, woraus abgeleitet werden kann wie viele Stützkörper 2 in den Knochen 4 gepresst wurden. Die Stützkörper 2 könnten auch in einer zusätzlichen Kanüle angeordnet sein, welche in die Kanüle 6 eingeführt wird, bevor die Pressvorrichtung 9 mit der Kanüle 6 verbunden wird. Somit wäre innerhalb der Kanüle 6 eine zweite Kanüle angeordnet, welche die Stützkörper 2 enthält. Derart könnte die Kanüle 6 sehr einfach mit den Stützkörpern 2 beschickt werden. Die die Stützkörper 2 enthaltende Kanüle muss einen derart der Grösse der Stützkörper 2 angepassten Innendurchmesser aufweisen, dass die Stützkörper 2 nur nacheinander folgend angeordnet und darin verschiebbar sind, um die Stützkörper 2 nacheinanderfolgend dem Knocheninnenraum 4a zuzuführen. Nach dem Füllen des Knocheninnenraumes 4a wird die Pressvorrichtung 9 und die Kanüle 6 entfernt und das Loch im Knochen, wie in Figur 4 dargestellt, mit einem Pfropfen 4c verschlossen.

Die Kanüle 6 kann nicht nur, wie in den Figuren 10 bis 13 dargestellt, einen runden Innenquerschnitt aufweisen, sondern auch andere Querschnittsformen, beispielsweise wie in Figur 15 dargestellt einen sechseckigen oder wie in Figur 16 dargestellt einen rechteckigen Innenquerschnitt, welche beispielsweise zum Einführen des in Figur 6a dargestellten Stützkörpers 2 geeignet sind. Die Kanüle 6 kann auch weitere, der jeweiligen Kontur des verwendeten Stützkörpers 2 angepasste Querschnittformen aufweisen.

Die Spitze 6a der Kanüle 6 kann auch ohne Befestigungsmittel 6c ausgestaltet sein. Insbesondere falls bereits vorgängig ein Knochenhohlraum 4b gebildet wurde, können die Stützkörper 2, wie in Figur 2 dargestellt, auch mit einer Spritzen ähnlichen Vorrichtung 9 dem Knochenhohlraum 4b zugeführt werden. In einem vorteilhaften Verfahren wird nacheinander folgend jeweils eine Gruppe von Stützkörpern, beispielsweise 5 oder 10 Stützkörper, dem Knochenhohlraum 4b zugeführt, danach die Kanüle 6 entfernt, und mit Hilfe eines Stopfwerkzeuges die sich im Knochenhohlraum 4b befindlichen Stützkörper 2 zusätzlich hineingepresst und verdichtet, um danach wieder die Kanüle am Knochen 4 anzusetzen, und eine weitere Gruppe von Stützkörpern 2 in den Knochenhohlraum 4b einzuführen, und diese wiederum mit dem Stopfwerkzeug zu verdichten.

In einem möglichen Verfahrensschritt kann, nachdem die Stützkörper 2 in den Knocheninnenraum 4a eingeführt wurden, eine osteoinduktive und/oder osteokonduktive Substanz zugeführt, beispielsweise als Flüssigkeit oder als Fluid, um die noch vorhandenen Hohlräume im Knocheninnenraum 4a mit dieser Substanz zu füllen. Das Füllmittel 1 könnte auch gemeinsam mit einem Fluid dem Knocheninnenraum 4a zugeführt werden, indem beispielsweise die Stützkörper 2 gemeinsam mit einem Fluid, insbesondere gemischt, in der Pressvorrichtung 9 zum Einführen in den Knocheninnenraum 4a bereitstehen.

## Patentansprüche

1. Trockenfliessfähiges Füllmittel (1) zum Ausbilden einer Stützstruktur in einem Knocheninnenraum (4a), umfassend eine Mehrzahl biokompatibler Stützkörper (2), welche unter den üblicherweise im Knocheninnenraum (4a) auftretenden physiologischen Lasten resistent gegen Verformung oder Bruch sind, wobei die Stützkörper (2) eine Grösse zwischen 2 mm und 10 mm aufweisen.

2. Füllmittel nach Anspruch 1, **dadurch gekennzeichnet, dass** der Stützkörper (2) einen offenen Innenhohlraum (3) aufweist.

3. Füllmittel nach Anspruch 2, **dadurch gekennzeichnet, dass** jeder Stützkörper (2) ein Gesamtvolumen beansprucht, welches das Volumen des Materials des Stützkörpers (2) sowie dessen Innenhohlraum (3) umfasst, wobei das Volumen des Innenhohlraumes (3) mehr als 30% des Gesamtvolumens beträgt.

4. Füllmittel nach Anspruch 3, **dadurch gekennzeichnet, dass** das Volumen des Innenhohlraumes (3) mehr als 50% des Gesamtvolumens beträgt.

5. Füllmittel nach einem der Ansprüche 2 bis 4, **dadurch gekennzeichnet, dass** der Innenhohlraum (3) zusammenhängend und nicht porös ausgestaltet ist.

6. Füllmittel nach einem der Ansprüche 2 bis 5, **dadurch gekennzeichnet, dass** der Innenhohlraum (3) zylinderförmig ausgestaltet ist.

7. Füllmittel nach einem der Ansprüche 2 bis 6, **dadurch gekennzeichnet, dass** die Stützkörper (2) ringförmig ausgestaltet sind.

8. Füllmittel nach einem der vorhergehenden Ansprüche, **dadurch gekennzeichnet, dass** alle Stützkörper (2) bezüglich Grösse und Form identisch ausgestaltet sind.

9. Füllmittel nach einem der vorhergehenden Ansprüche, **dadurch gekennzeichnet, dass** die Stützkörper (2) eine polyederförmige Aussenkontur aufweisen.

10. Füllmittel nach einem der vorhergehenden Ansprüche, **dadurch gekennzeichnet, dass** die Stützkörper (2) eine eckige, insbesondere 3- bis 10-eckige Aussenkontur und vorzugsweise eine 4-, 5- oder 6-eckige Aussenkontur aufweisen.

11. Füllmittel nach einem der Ansprüche 1 bis 7, **dadurch gekennzeichnet, dass** die Stützkörper (2) eine sphärische Aussenkontur aufweisen, insbesondere eine kugelförmige oder eine ellipsoidförmige Aussenkontur.

12. Füllmittel nach einem der vorhergehenden Ansprüche, **dadurch gekennzeichnet, dass** der Stützkörper (2) einen Aussendurchmesser (D) und orthogonal dazu eine Höhe (H) aufweist, und dass der Aussendurchmesser (D) zumindest das 1,5-fache der Höhe (H) beträgt.

13. Füllmittel nach einem der vorhergehenden Ansprüche, **dadurch gekennzeichnet, dass** die Stützkörper (2) eine derart geformte Oberfläche aufweisen, dass zwischen den im Knocheninnenraum (4a) eingefüllten Stützkörpern (2) ein gegenseitiges Verkeilen auftritt, um innerhalb des Knochenhohlraums (4a) eine zusammenhängende Stützstruktur zu bilden.

14. Füllmittel nach Anspruch 13, **dadurch gekennzeichnet, dass** die Oberfläche der Stützkörper (2) Formen aus der Gruppe Ecke, Kante, Spitze, Vertiefung, Durchbrechung oder eine Kombination davon aufweisen, wobei diese Formen derart gegenseitig angepasst ausgestaltet sind, dass sich die Stützkörper gegenseitig verkeilen können.

15. Füllmittel nach einem der Ansprüche 13 bis 14, **dadurch gekennzeichnet, dass** der Stützkörper (2) eine einer Schraubenmutter ähnliche Form aufweist, mit einer mehreckigen Aussenkontur und einem im wesentlichen zylinderförmigen Innenhohlraum (3).

16. Füllmittel nach einem der vorhergehenden Ansprüche, **dadurch gekennzeichnet, dass** die Stützkörper (2) bestehen aus:
- keramischen Werkstoffen, insbesondere Calciumphosphate /Hydroxylapatite, Aluminiumoxide, Zirkoniumoxide, ATZ Geramk (Alu-Zirkonium-Oxid), bioaktive Gläser, Glaskeramiken, Porzellan oder einer Kombination davon, oder
- metallischen Werkstoffen, insbesondere Titan, Tantal, rostfreier Stahl, Stahllegierungen wie Kobalt-Chrom Legierung, Titanlegierungen wie Titan-Nickel Legierung oder Titan-Aluminium-Niobium/Vanadium Legierung, oder einer Kombination davon, oder
- Polymeren, insbesondere Polymethylmethacryl (PMMA), Polyetheretherketon (PEEK) Polyethylen (PE), Polyethylenterephthalat (PET), oder einer Kombination davon, oder
- biodegradablen Polymeren wie Polylactate.

17. Füllmittel nach einem der vorhergehenden Ansprüche, **dadurch gekennzeichnet, dass** der Innenhohlraum (3) mit zumindest eine osteoinduktive und/oder osteokonduktiven Substanz gefüllt ist, insbesondere knochenwachstumsförderndes Protein, Kalziumsulfat, oder eine Kombination davon.

18. Zuführvorrichtung (5) zum Einführen eines trockenfliessfähigen Füllmittels (1) in einen Knocheninnenraum (4a), wobei das Füllmittel (1) aus biokompatiblen Stützkörpern (2) mit einer Grösse zwischen 2 mm und 10 mm besteht, wobei die Zuführvorrichtung (5) eine Kanüle (6) umfasst, mit einem derart der Grösse der Füllmittel (1) angepassten Innendurchmesser, dass die Stützkörper (2) nur nacheinander folgend dem Knocheninnenraum (4a) zuführbar sind.

19. Zuführvorrichtung nach Anspruch 18, **dadurch gekennzeichnet, dass** die Kanüle (6) ein freies Ende (6a) mit einem Befestigungsmittel (6c) aufweist, wobei das Befestigungsmittel (6c) derart ausgestaltet ist, dass dieses lösbar mit dem Knochen (4) verbindbar ist.

20. Zuführmittel nach einem der Ansprüche 18 bis 19, **dadurch gekennzeichnet, dass** dieses ein Mittel (9) umfasst, welches in Förderrichtung der Stützkörper (2) einen Druck auf diese ausübt, um die Stützkörper (2) unter Druck dem Knochen (4) zuzuführen.

21. Verfahren zum Füllen eines Knocheninnenraumes (4a), insbesondere eines Wirbelkörpers, mit einer Stützstruktur, indem eine Mehrzahl biokompatibler Stützkörper (2), welche unter den üblicherweise im Knocheninnenraum (4a) auftretenden physiologischen Lasten resistent gegen Verformung oder Bruch sind, nacheinanderfolgend den Knocheninnenraum (4a) zugeführt werden, und die Stützkörper (2) bezüglich ihrer Lage willkürlich innerhalb des Knocheninnenraumes (4a) angeordnet werden.

22. Verfahren nach Anspruch 21, **dadurch gekennzeichnet, dass** vorerst im Knocheninnenraum (4a) ein Knochenhohlraum (4b) geschaffen wird, und danach die Stützkörper (2) dem Knochenhohlraum (4b) zugeführt werden.

23. Verfahren nach Anspruch 21 oder 22, **dadurch gekennzeichnet, dass** die Stützkörper (2) derart unter Druck dem Knocheninnenraum (4a) zugeführt werden, dass die Stützkörper (2a) einen Knochenhohlraum (4b) ausbilden oder diesen erweitern.

24. Verfahren nach einem der Ansprüche 21 bis 23, **dadurch gekennzeichnet, dass** mehrmals nacheinanderfolg vorerst eine Gruppe von Stützkörpern (2) dem Knocheninnenraum (4a) zugeführt werden, und danach die sich im Knocheninnenraum (4a) befindlichen Stützkörper (2) mit einem Instrument gestopft werden.

25. Verfahren nach einem der Ansprüche 21 bis 24, **dadurch gekennzeichnet, dass** die Stützkörper (2) einen Innenhohlraum (3) aufweisen, und dass dieser Innenhohlraum (3) vor dem Einführen der Stützkörper (2) mit einer osteoinduktiven und/oder osteokonduktiven Substanz gefüllt wird.

26. Verfahren nach einem der Ansprüche 21 bis 24, **dadurch gekennzeichnet, dass** dem Knocheninnenraum (4a), nachdem alle Stützkörper (2) eingeführt wurde, eine osteoinduktive und/oder osteokonduktive Substanz zugeführt wird, insbesondere ein Fluid.
